# EUROPEAN PATENT SPECIFICATION

(11) **EP 3 079 557 B1**
(45) Date of publication and mention of the grant of the patent: **17.04.2019**
(21) Application number: 14870076.8
(22) Date of filing: 11.12.2014
(51) Int. Cl.: A61B 1/06, A61B 1/317, A61B 1/04, A61B 1/055

(54) **MEDICAL IMAGING DEVICE USING THERMALLY CONDUCTING LENS CRADLE**
MEDIZINISCHE BILDGEBUNGSVORRICHTUNG MIT WÄRMELEITENDEM LINSENTRÄGER
DISPOSITIF D'IMAGERIE MÉDICALE UTILISANT UN BERCEAU THERMOCONDUCTEUR À PLUSIEURS LENTILLES

(30) Priority: 13.12.2013 US 201361916043 P
(43) Date of publication of application: 19.10.2016
(73) Proprietor: Integrated Endoscopy Inc., Rancho Santa Margarita, CA 92688 (US)
(72) Inventor: HOYLE, Lonnie R., Rancho Santa Margarita, CA 92688 (US); ALMARZOUK, Kais, Rancho Santa Margarita, CA 92688 (US)
(74) Representative: Eidelsberg, Olivier Nathan
(86) International application number: PCT/US2014/069842
(87) International publication number: WO 2015/089330

(56) References cited:
- WO-A2-2004/036266
- US-A1- 2006 258 902
- US-A1- 2008 062 540
- US-A1- 2009 018 397
- US-A1- 2009 219 713
- US-A1- 2011 263 941
- US-A1- 2011 263 941
- US-A1- 2012 029 289
- US-A1- 2012 176 669
- US-B1- 6 424 473

## Description

### BACKGROUND

Various embodiments herein relate generally to endoscopes, arthroscopes, and other medical imaging devices. Endoscopes can be inserted into the body to form images of features within a cavity therein. Endoscopes generally include an elongate tubular structure that includes optics for imaging. Endoscopes may additionally be configured to provide illumination. Since endoscopes can provide images of within the patient's body, endoscopes are useful diagnostic and/or surgical tools.

### SUMMARY

The systems, methods and devices of this disclosure each have several innovative aspects, no single one of which is solely responsible for the desirable attributes disclosed herein. The invention is defined in independent claim 1 and the dependent claims 2 - 11.

Various innovative aspects of the subject matter described in this disclosure can be implemented in the following embodiments:
Embodiment 1: An endoscope, comprising:
   a proximal end portion;
   a distal end portion;
   a plurality of lenses disposed in an optical path extending from said distal end portion to said proximal end portion such that an image of an object at the distal end portion can be formed at the proximal end portion;
   at least one solid state emitter disposed at the distal end portion, the at least one solid state emitter generating heat when activated; and
   a thermally conductive cradle forming an elongate inner open region configured to house said plurality of lenses, said thermally conductive cradle attached to the distal end portion, the thermally conductive cradle configured to dissipate heat generated by the at least one solid state emitter away from the distal end portion,
   wherein the thermally conductive cradle comprises copper.
Embodiment 2: The endoscope of Embodiment 1, wherein said endoscope comprises an arthroscope.
Embodiment 3: The endoscope of any of Embodiments 1 - 2, wherein the at least one solid state emitter comprises at least one light emitting diode.
Embodiment 4: The endoscope of any of Embodiments 1 - 3, wherein said plurality of lenses comprise rod lenses having lengths and widths, said lengths being longer than said widths.
Embodiment 5: The endoscope of any of Embodiments 1 - 4, wherein the thermally conductive cradle comprises separate first and second sections, wherein the first section and second section comprise portions of a right circular cylinder physically separated by a gap along a longitudinal length of the right circular cylinder.
Embodiment 6: The endoscope of Embodiment 5, wherein the first and second sections have substantially equal size.
Embodiment 7: The endoscope of any of Embodiments 5 - 6, wherein the first and second sections each comprise substantially a half of said right circular cylinder.
Embodiment 8: The endoscope of any of Embodiments 5 - 7, wherein the first half and the second half each has two longitudinal edges, and wherein a first longitudinal edge of the first half and a first longitudinal edge of the second half are separated by a space at least 0.5 millimeter.
Embodiment 9: The endoscope of any of Embodiments 5 - 8, wherein a second longitudinal edge of the first half and a second longitudinal edge of the second half are physically separated by a space.
Embodiment 10: The endoscope of any of Embodiments 5 - 9, wherein a second longitudinal edge of the first half and a second longitudinal edge of the second half are physically spaced apart by a spacer.
Embodiment 11: The endoscope of any of Embodiments 5 -10, further comprising:
   a first electrical line connected to a cathode of one of the at least one solid state emitters; and
   a second electrical line connected to an anode of one of the at least one solid state emitter,
   wherein the first and second electrical lines are disposed in the space between said first and second sections.
Embodiment 12: The endoscope of Embodiment 11, wherein the first and second electrical lines comprise electrical wires.
Embodiment 13: The endoscope of any of Embodiments 1 - 12, wherein the thermally conductive cradle is electrically conductive and said the thermally conductive cradle provides at least one electrical path to the at least one solid state emitter.
Embodiment 14: The endoscope of any of Embodiments 1 - 13, wherein the first section is electrically connected to a cathode on at least one of said at least one solid state emitter and the second section is electrically connected to an anode on at least one of said at least one solid state emitter.
Embodiment 15: The endoscope of any of Embodiments 1 -14, further comprising an outer tube surrounding the thermally conductive cradle.
Embodiment 16: The endoscope of any of Embodiments 1 - 15, wherein said cradle is electrically insulated from said outer tube.
Embodiment 17: The endoscope of any of Embodiments 1 - 16, wherein the distal end comprises a passageway forming a portion of the optical path, wherein the passageway comprises first and second reflective surfaces, such that the portion of the optical path through the passageway does not include a glass optical element.
Embodiment 18: The endoscope of any of Embodiments 1 - 18, wherein the first and the second reflective surfaces comprise metallized substrates.
Embodiment 19: The endoscope of Embodiment 18, wherein the substrates include glass.
Embodiment 20: The endoscope of any of Embodiments 18 - 19, wherein inner surfaces of the substrates that are adjacent to the passageway are metallized.
Embodiment 21: The endoscope of any of Embodiments 1 - 20, wherein the at least one solid state emitter is disposed on a base.
Embodiment 22: The endoscope of Embodiment 21, wherein the base comprises a thermally conducting material.
Embodiment 23: The endoscope of any of Embodiments 21 - 22, wherein the base comprises a ceramic material.
Embodiment 24: The endoscope of any of Embodiments 21 - 23, wherein the base comprises electrically conducting pathways configured to provide electrical power to the at least one solid state emitter.
Embodiment 25: The endoscope of any of Embodiments 21 - 24, wherein at least a portion of the base is metallized.
Embodiment 26: An endoscope, comprising:
   a proximal end portion;
   a distal end portion;
   at least one solid state emitter disposed at the distal end portion, the at least one solid state emitter generating heat when activated;
   a cradle attached to the distal end portion, wherein the cradle is thermally conductive and configured to dissipate heat generated by the at least one solid state emitter away from the distal end portion, wherein the cradle is further electrically conductive and is configured to provide an electrical path to the at least one solid state emitter; and
   at least one lens disposed inside the cradle.
Embodiment 27: The endoscope of Embodiment 26, wherein the endoscope comprises an arthroscope.
Embodiment 28: The endoscope of any of Embodiments 26 - 27, wherein the at least one solid state emitter comprises at least one light emitting diode.
Embodiment 29: The endoscope of any of Embodiments 26 - 28, wherein the at least one lens comprises a rod lens having a length and a width, said length being longer than said width.
Embodiment 30: The endoscope of any of Embodiments 26 - 30, wherein the cradle comprises copper.
Embodiment 31: The endoscope of any of Embodiments 26 - 31, further comprising an outer tube surrounding the thermally conductive cradle.
Embodiment 32: The endoscope of Embodiment 31, wherein the cradle is electrically insulated from said outer tube.
Embodiment 33: The endoscope of any of Embodiments 26 - 32, wherein the distal end comprises a passageway forming a portion of the optical path, wherein the passageway comprises first and second reflective surfaces, such that the portion of the optical path through the passageway does not include a glass optical element.
Embodiment 34: The endoscope of Embodiment 33, wherein the first and the second reflective surfaces comprise metallized substrates.
Embodiment 35: The endoscope of any of Embodiments 33 - 34, wherein the substrates include glass.
Embodiment 36: The endoscope of any of Embodiments 33 - 35, wherein inner surfaces of the substrates that are adjacent to the passageway are metallized.
Embodiment 37: The endoscope of any of Embodiments 26 - 36, wherein the at least one solid state emitter is disposed on a base.
Embodiment 38: The endoscope of any Embodiment 37, wherein the base comprises a thermally conducting material.
Embodiment 39: The endoscope of any of Embodiments 37 - 38, wherein the base comprises a ceramic material.
Embodiment 40: The endoscope of any of Embodiments 37 - 39, wherein the base comprises electrically conducting pathways configured to provide electrical power to the at least one solid state emitter.
Embodiment 41: The endoscope of any of Embodiments 37 - 40, wherein at least a portion of the base is metallized.
Embodiment 42: A method of using an endoscope, the method comprising:
   causing at least one solid state emitter to generate heat, wherein the at least one solid state emitter is disposed on a distal end portion of the endoscope; and
   dissipating heat generated by the at least one solid state emitter away from the distal end portion via a thermally conductive cradle attached to the distal end portion,
   wherein the thermally conductive cradle comprises copper.
Embodiment 43: The method of Embodiment 42, wherein the thermally conductive cradle comprises separate first and second sections, wherein the first section and the second section comprise portions a right circular cylinder physically separated by a gap along a longitudinal length of the right circular cylinder.
Embodiment 44: The method of any of Embodiments 42 - 43, wherein the thermally conductive cradle is further electrically conductive, and wherein the method further comprises providing electricity to the at least one solid state emitter via the thermally conductive cradle.
Embodiment 45: The method of any of Embodiments 42 - 44, wherein the endoscope comprises at least one rod lens disposed inside the thermally conductive cradle.
Embodiment 46: An endoscope, comprising:
   a proximal end portion;
   a distal end portion;
   a plurality of lenses disposed in an optical path extending from said distal end portion to said proximal end portion such that an image of an object at the distal end portion can be formed at the proximal end portion;
   at least one solid state emitter disposed at the distal end portion, the at least one solid state emitter generating heat when activated; and
   a thermally conductive cradle comprising an elongate inner open region configured to house said plurality of lenses, said thermally conductive cradle attached to the distal end portion,
   wherein the thermally conductive cradle comprises separate first and second sections, wherein the first section and the second section comprise portions a right circular cylinder physically separated by a gap along a longitudinal length of the right circular cylinder.
Embodiment 47: The endoscope of Embodiment 46, wherein the first and second sections have substantially equal size.
Embodiment 48: The endoscope of any of Embodiments 46 - 47, wherein the first and second sections each comprise substantially a half of said right circular cylinder.
Embodiment 49: The endoscope of Embodiment 48, wherein the first half and the second half each has two longitudinal edges, and wherein a first longitudinal edge of the first half and a first longitudinal edge of the second half are separated by a space at least 0.5 millimeter.
Embodiment 50: The endoscope of Embodiment 49, wherein a second longitudinal edge of the first half and a second longitudinal edge of the second half are physically spaced apart by a spacer.
Embodiment 51: The endoscope of any of Embodiments 49 - 50, wherein a second longitudinal edge of the first half and a second longitudinal edge of the second half are physically separated by a space.
Embodiment 52: The endoscope of any of Embodiments 46 - 51, further comprising:
   a first electrical line connected to a cathode of one of the at least one solid state emitters; and
   a second electrical line connected to an anode of one of the at least one solid state emitters,
   wherein the first and second electrical lines are disposed in a space between said first and second sections.
Embodiment 53: The endoscope of any of Embodiments 46 - 52, wherein said endoscope comprises an arthroscope.

Details of one or more implementations of the subject matter described in this disclosure are set forth in the accompanying drawings and the description below. Although the examples provided in this disclosure are primarily described in terms of a medical imaging device, the concepts provided herein may apply to other types of imaging systems and devices. Other features, aspects, and advantages will become apparent from the description, the drawings and the claims. Note that the relative dimensions of the following figures may not be drawn to scale.

### BRIEF DESCRIPTION OF THE DRAWINGS

Fig. 1 illustrates a schematic perspective view of an endoscope, (e.g., an arthroscope), including a plurality of lenses supported within and by a lens cradle according to various embodiments disclosed herein.
Fig. 1A & Fig. 1B illustrate schematic cross-sectional views of an "air prism" according to various embodiments disclosed herein.
Fig. 2 illustrates a schematic exploded perspective view of an endoscope cradle comprising two portions or halves according to various embodiments disclosed herein.
Fig. 3 illustrates a schematic view of the distal end portion of an endoscope showing the lens cradle as well as a solid state light emitter coupled to electrical lines disposed between two halves of the cradle.
Fig. 4 illustrates a schematic exploded perspective view of a unitary cradle for supporting a plurality of rod lenses and an outer tube configured to surround the cradle and lenses.

### DETAILED DESCRIPTION OF VARIOUS EMBODIMENTS

Some embodiments herein comprise endoscopes for viewing inside a cavity of a body. Various embodiments of the endoscopes comprise a plurality of lenses disposed within an elongate tubular structure having proximal and distal ends. These the lenses can relay an image of features in the body located at the distal end of the endoscope to the proximal end of the endoscope. In some embodiments, a detector such as a two-dimensional CCD or CMOS detector array can be included at the proximal end of the endoscope to sense the relayed image. In some embodiments, an eyepiece or other optics may be used to view the image. In certain embodiments, the endoscope may additionally have a light source that is configured, sized, and positioned so as to be inserted into the body cavity to provide illumination therein. In some embodiments, for example, this light source is disposed at the distal end of the endoscope. In various embodiments, this light source comprises at least one solid state emitter, such as a light emitting diode (LED), located at the distal end of the endoscope. One challenge of such a configuration is that the solid state emitters such as LEDs may generate heat as they emit light

Accordingly, various embodiments disclosed herein are designed to conduct heat away from the LED at the distal end of the endoscope. One approach described herein is to employ a structure such as a cradle for holding the plurality of lenses that also is a conduit for extracting heat from the distal end of the endoscope where the LED is located. In some embodiments, for example, an endoscope may include a thermally conductive cradle or lens holder for holding the plurality of lenses that extends to the distal end of the endoscope. The thermally conductive cradle may comprise a highly thermally conductive material such as copper, aluminum, etc. The thermally conductive cradle may therefore be capable of conducting heat away from the distal end of the endoscope resulting in a lower temperature at the distal end of the endoscope or of the LED or other components at the distal end. In certain embodiments disclosed herein, the thermally conductive cradle may also be electrically conductive.

In operation, light emitted from the light source illuminate and is reflected off objects, surfaces, and features (e.g., walls) in the interior of the body cavity. A portion of the reflected light may be collected through an aperture at the distal end of the endoscope. This light may be directed along an optical path through the endoscope formed by the plurality of lenses disposed in the cradle so as to form an image of the objects, surfaces, features at the proximal end of the endoscope. In certain embodiments, the series of lenses can comprise rod lenses disposed inside the thermally conductive cradle. The light collected may then be directed to an optical sensor such as, for example, an optical detector array or an optical camera. Thus, an image of the object, surface, feature, etc. inside the body cavity can be viewed, for example, by the physician possibly on a display in communication with the detector.

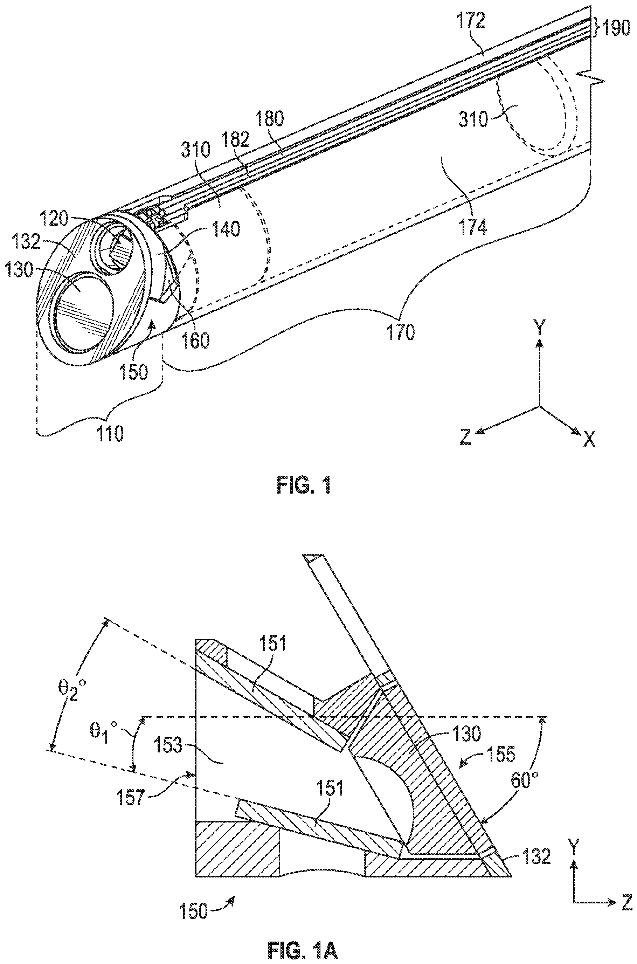

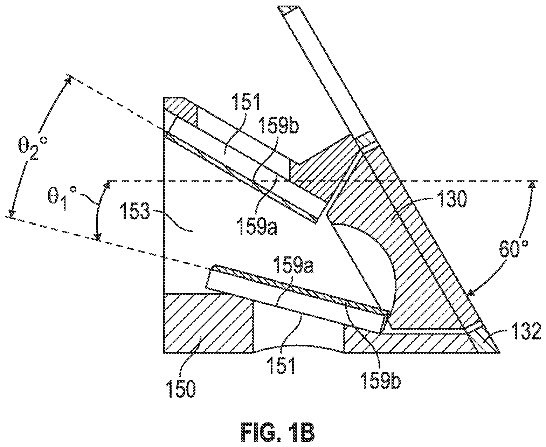

For illustrative purposes, Fig. 1 illustrates a portion of an endoscope such as, for example, an arthroscope configured to conduct heat from the distal end thereof. The endoscope comprises an elongate member that may be inserted into a portion of a body such as a human body. Additional discussion of endoscopes is U.S. Patent No. 7,976,462.

Referring to Fig. 1, the endoscope includes a distal end portion 110 and a lens cradle 170 attached to the distal end portion. The distal end portion 110 may include a solid state emitter 120. The solid state emitter 120 may be configured to radiate light and illuminate internal regions of the body. Although Fig. 1 illustrates one solid state emitter, in other embodiments a plurality of solid state emitters may be disposed at the distal end portion 110 of the endoscope, such as for example similar to the embodiments disclosed in U.S. Patent No. 7,976,462. In some embodiments, the solid state emitter 120 emits white light, although the emitter 120 does not need to be a white light emitter. A colored emitter that radiates in narrow wavelength ranges may be employed as well. For example, images may be formed by optical sensors that are sensitive to the particular wavelength region used for illumination. In certain embodiments, a specific wavelength illumination may be employed for fluorescence applications. Accordingly, the emitter may emit broad or narrow band ultraviolet as well as infrared light.

Referring to Fig. 1, the distal end portion 110 may include an air prism block 150. Fig. 1A shows a cross-sectional view of such an air prism and air prism block 150. The air prism block 150 comprises a body having an input aperture 155, a passageway 153 through the body, and an output aperture 157. The body 150 includes internal sidewalls that define the passageway 153 through the body. Reflectors 151 may be disposed in the passageway to assist the propagation of light therethrough. The embodiment shown includes two such reflectors 151. The reflectors 151 can be disposed at an angle θ₂ with respect to each other as shown in Fig. 1A. The angle θ₂ between the reflectors 151 can depend on the offset angle of the endoscope. For example, for a 30-degree offset endoscope, the angle θ₂ can be about 15 degrees. As another example, for a 75-degree offset endoscope, the angle θ₂ can be about 37.5 degrees. The reflectors 151 can also be angled with respect to a central axis of the endoscope. For example, as shown in Fig. 1A, one of the reflectors 151 is disposed at an angle θ₁ with respect to the central axis of the endoscope. Without any loss of generality, in various embodiments, the angle θ₁ can be equal to the angle θ₂.

The reflectors 151 may comprise mirrors (as illustrated) and/or metallization for example on the sidewalls of the air prism block 150 that reflects light. In some embodiments, for example, mirrors comprising metalized glass plates are disposed on the internal sidewalls of the passageway 153. In such cases, light received through the input aperture 155 propagate through the passageway 153 being reflected off reflective surfaces of the reflectors 151 to the output aperture 157. Fig. 1B shows a cross-sectional view of an embodiment of an air prism including mirrors comprising a pair of metalized substrates. The substrates can comprise glass, ceramic, plastic, acrylic or any material that can provide sufficient structural support The substrates have a first surface 159a adjacent the body 150 and a second surface 159b opposite the first surface 159a. The second surface 159b forms a portion of the boundary of the passageway 153. As illustrated in Fig. 1B, the second surface 159b of the substrate is metallized such that light collected at the distal end of the endoscope travels through the passageway by multiple reflections at the second surface 159b of the substrates towards the optical sensor. Metallizing the surface of the substrates adjacent the passageway can advantageously reduce or eliminate the amount of dispersion between the various wavelengths of light that can result when travelling through the material of the substrate. In some embodiments, the body 150 may comprise plastic, ceramic, or metal. In some embodiment the body 150 comprises thermal conducting material such as metal or thermally conducting ceramic. Various implementations of an endoscope including an air prism are also described in U.S. Patent No. 7,976,462.

Referring again to Fig. 1, the distal end portion 110 additionally comprises a front lens 130 and a cover plate 132. The front lens 130 is disposed in the input aperture 155 of the air prism block 150. The cover plate 132 is disposed over (or forward) the front lens 130 in front of the air prism block 150. The cover plate 132 includes a hole therein that is disposed with respect to the front lens 130 such that light can pass through the hole and to the front lens. Accordingly, the cover plate 132 is disposed on the air prism block 150 in a manner to align the hole in the cover plate with the front lens 130. Accordingly, light received by the front lens 130 can propagate into the input aperture 155 of the air prism 150 and therethrough. The cover plate 132 may comprise metal such as stainless steel in some embodiments, although other materials may be used.

The cover plate 132 also has a hole therein that is disposed with respect to the solid state emitter 120 to permit light to from the emitter to pass through the cover plate.

In various embodiments the front lens 130 comprises glass, sapphire, or other substantially optically transmissive or transparent material. In some embodiments, the front lens 130 has negative power. In certain embodiments, the front lens 130 comprises a plano-concave lens with a plano surface disposed outward (e.g., more distal). Accordingly, in various embodiments the front lens 130 collects light reflected from surfaces and/or features within the body cavity and this light propagates into the air prism, being reflected of the reflective surfaces therein, so as to exit the output aperture 157 of the air prism. The front lens 130 may provide for increased field of view and may be tilted at an angle with respect to the longitudinal direction (e.g., z-axis) so as to image sidewalls of the body cavity. Moreover, in some embodiments, the front surface of the distal end portion 110 may be angled so that light can be collected at the distal end of the endoscope from an oblique direction with respect to the endoscope. For example, the endoscope may be used to observe an inner side wall of a cavity in the body by inserting the endoscope into the cavity and rotating the endoscope such that the angled front surface is directed toward a portion of the inner side wall of the cavity. Other designs are possible.

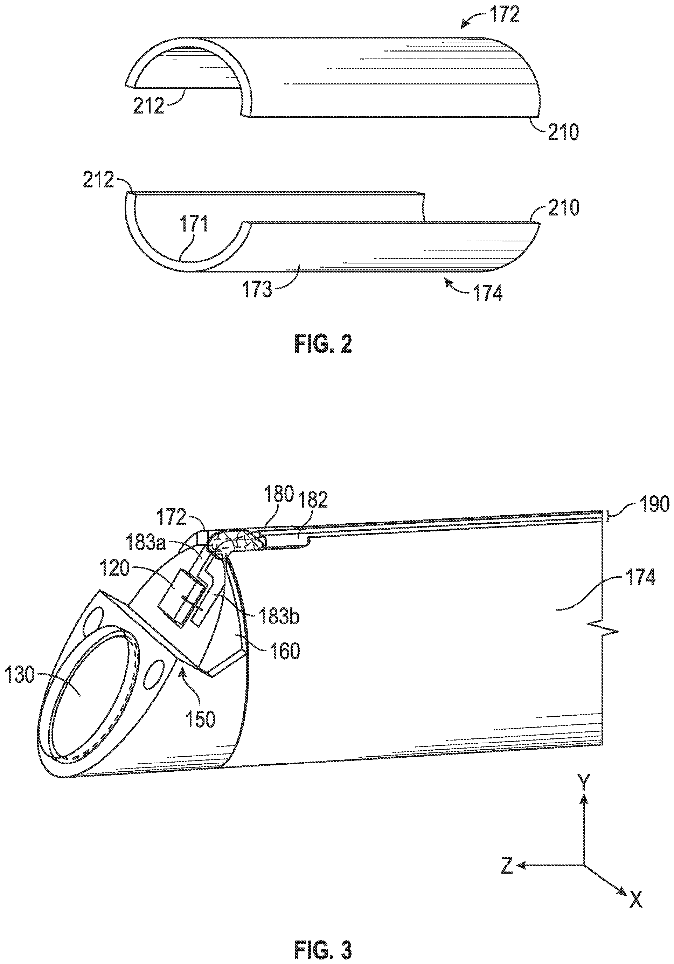

The solid state emitter 120 may be mounted on a base 160. Fig. 3 shows an additional view of this base. In various embodiments the base 160 comprises thermal conducting material. In various embodiments, this base 160 comprises electrically insulating material. In certain embodiments, this base 160 comprises a thermal conducting insulator such as a ceramic. In various implementations, the base 160 can comprise materials, such as, for example, alumina (e.g. Al₂O₃) and/or nitride (e.g., silicon nitride). In addition, in certain embodiments, the base 160 may be coated with a coating to enhance the thermal conducting properties of the base 160. For example, the base 160 may be metalized such as with gold coating in order to provide the base 160 with thermally conductive and/or electrically conductive properties.

In various embodiments, the base 160 is configured to receive the emitter (e.g., LED) 120. For example, the base 160 may include holes for receiving pins disposed on the LED 120. These pins may comprise electrical connections to the emitter 120, for example, an anode and a cathode of a light emitting diode. The base 160 may be metallized and/or comprise metal pathways to provide electrical connections to the cathode and anode of the solid state emitter 120. These pathways may be formed from patterned metallization such as patterned gold. The base 160 is disposed in part on the air prism block 150. Further, as illustrated in Fig. 3, the base 160 may be in contact with the cradle 170 possibly over a large area. In various embodiments, this interface is a sufficient thermal contact to effectively transfers heat from the LED base 160 to the cradle 170. Accordingly, the base 160, being a strong thermal conductor, may assist in dissipating heat generated from the solid state emitter 120 away from the distal portion 110 of the endoscope for example, via the cradle 170 as discussed below.

As illustrated in Fig. 1, the distal end portion 110 further comprises a reflector 140. The reflector 140, in the embodiment shown, comprises a plate having an aperture therein. The aperture has a perimeter defined by a wall or surface. This surface may be at least partially reflecting and may be white or other reflecting color and/or may comprise reflective material such as a metal coating to reflect light. The plate 140 is disposed on the LED base 160 such that the LED 120 is within the aperture and framed by the reflecting surface. So configured, at least a portion of the light emitted in lateral and/or reward directions from the emitter may possibly be reflected forward. In various embodiments, the reflecting surface can have a shape or contour to enhance useful reflection. Accordingly, in various embodiments, the reflector 140 may be configured to reflect and redirect light so as to avoid wasting light. In some embodiments, the reflector plate 140 comprises thermal conducting material such as metal or thermally conducting ceramic. In various embodiments, the reflector plate 140 is in good thermal contact with the base 160.

As illustrated in Figs. 1 and 3 and referenced above, the distal end portion 110 is connected to the lens cradle 170. In particular, in the embodiments below, the LED base 160 (and possibly the air prism block 150) are firmly attached to the distal end of the cradle 170 so as to provide a sufficiently good thermal contact to readily transfer heat from the LED base (and possibly the air prism block) to the cradle. As shown in Fig. 1, the lens cradle 170 provides an elongate support for one or more optical elements lenses 310. For example, the cradle 170 may surround, at least partially, rod lenses. Accordingly, the cradle 170 contains a central inner open region for housing the plurality of lenses 310. In various embodiments, this central inner open region is shaped such that the lenses 310, which may have cylindrical edges, fit conformally therein.

Accordingly, in various embodiments, the cradle 170 may comprise a hollow cylindrical tube or portions thereof. The hollow cylindrical tube may provide an optical path for propagating light from the distal end portion 110 of the endoscope to the proximal end of the endoscope. The cradle 170 may be a small, e.g., narrow, structure for non-invasive insertion into the body. In various embodiments, for example, the cradle 170 has a width of less than 5 mm or 4 mm but greater than 1 mm or 2 mm. In various embodiments the width or outer diameter is between about 3-4 mm. The walls of the cradle 170 may be thin to allow for lenses 310 having sufficient aperture size to be disposed in the central inner cavity region of the cradle, maintaining a small cross-section for the endoscope. In some embodiments, for example, the walls are less than 1 mm or 0.5 mm thick but may be larger than 0.1 mm thick in various embodiments. In some embodiments, the walls are between 0.2 and 0.3 mm thick. The cradle 170 may be substantially longer than wide. For example, the length of the cradle 170 (e.g. in the z direction) may be between 100 mm and 400 mm and between 200 mm and 300 mm and may be between 10, 20, 30, 40, 50 times or more the width thereof but less than 100, 90, 80, 70, or 60 times the width thereof in some embodiments.

Referring to Figs. 1 and 2, in some embodiments, the cradle 170 comprises physically separate portions 172, 174 in the shape of a right circular cylinder split down its length, the longitudinal z-direction as shown. The separate components 172, 174, when assembled, may form or substantially form a right circular cylinder having the central open inner region for housing the lenses 310. In the embodiment shown in Figs. 1 and 2, the cradle 170 is split into two physically separate components 172, 174, e.g., two halves 172, 174, in the shape of a right circular cylinder cut in half down its length. The separate portions (e.g., halves) 172, 174 may be substantially equal in size in some embodiments, although the sizes of the separate portions (e.g. two halves) may be different in others. Similar, the two components 172, 174 when put together, need not completely produce a right circular cylinder. As discussed below, for example, spaces may exist between the two halves 172, 174 when assembled to support the lenses 310 in the endoscope.

Referring to Fig. 2, each halve 172, 174, includes has a concave inner surface 171 and a convex outer surface 173. The concave inner surface 171 is shaped to conformally fit the plurality of the lens 310 within the cradle 170 when the two halves 172, 174 are arranged together. Although the outer surface 173 is shown as convex, other shapes are possible. However, as discussed above, in various embodiments the cradle 170 is not very thick and thus a convex shaped outer surface 173 may likely accompany the concave inner surface 171.

Referring to Fig. 2, each half 172, 174 may include longitudinal edges 210, 212. The two halves 172, 174 may be arranged with respect to each other such that the longitudinal edges of the two halves are in proximity to each other. Referring to Fig. 1, the two halves 172, 174 may be separated by a space 190 disposed between the longitudinal edges 210, 212. In the embodiments shown, each of the two halves 172, 174 includes two longitudinal edges 210, 212. When the two halves 172, 174 are disposed together to form the assembled cradle 170, each of the longitudinal edges 210, 212 of one half 172, 174 may be separated from the longitudinal edges of the other half 172, 174 by the pair of spaces 190. In some embodiments discussed more fully below, the two halves 172, 174 may be physically separated so as to electrically isolate the two halves 172, 174. However, in some embodiments one or both of the spaces 190 are not present and the two halves 172, 174 touch each other instead of having a space therebetween. In certain embodiments, for example, one space is included between longitudinal edges 210 of respective halves whereas the other longitudinal edges 212 of the respective edges touch each other. In various embodiments, the space 190 (whether one or two) is greater than 0.1, 0.2, 0.3, 0.4, 0.5, 0.6, 0.8, 0.9, and 1.0 millimeters and smaller than 5 mm, 4 mm, 3 mm, 2 mm, 1 mm, 0.8 mm, or 0.6 mm. For example, in some embodiments at least one of the spaces is greater than 0.4 mm and less than 0.6 mm. In some embodiments, at least one of the spaces is greater than 0.9 mm and less than 2.0 mm. Other ranges are possible.

In various embodiments, the inner surface 171 of the cradle 170 is smooth and does not include surface features on the inner surface to space the lenses 310 from each other. Such a design, simplifies the manufacture of the cradle 170. For example, the cradle 170 or the separate halves 172, 174 of the cradle can be extruded in some embodiments. To space the lenses 310 apart, the lenses are appropriately placed with the proper longitudinal spacing therebetween in the cradle 170 or the cradle portions 172, 174 are disposed about the appropriately space lenses. Robotics may be employed to place the lenses 310 with the appropriate spacing between the lenses. In other, other embodiments, however, surface features may be include on the inner surface 171 of the cradle 170 or spacers may be included between the lenses 310.

As discussed above, in various embodiments, the solid state emitter 120 may generate substantial heat Accordingly, referring to Fig. 1, in some embodiments the cradle 170 may substantially entirely comprise a thermally conductive material, such as copper or aluminum. In certain embodiments, for example, the cradle 170 comprises at least 90%, 95% or more and up to 99% or 100% copper. Similarly, in certain embodiments, the cradle comprises at least 90%, 95% or more and up to 99% or 100% aluminum. In certain embodiments, the cradle 170 comprises at least 90%, 95% or more and up to 99% or 100% other highly conductive material such as a copper alloy or other metal. Forming the cradle 170 of highly thermally conductive material can enable heat generated by the solid state emitter 120 to be dissipated away from the distal end portion 110 of the endoscope where the emitter 120 is disposed. By transferring heat away from the distal end portion 110 of the endoscope, the thermally conductive cradle 170 advantageously allows the use of bright solid state emitters 120 that may generate significant heat without excessive heating at the distal end of the endoscope. Accordingly, for some embodiment, during use, the thermally conductive cradle 170 may allow the endoscope to remain within five degrees of body temperature.

In various embodiments, the plurality of lenses 310 comprises rod lenses. The rod lenses may be disposed in the inner central open region of the cradle, for example, formed when the two halves 172, 174 are assembled with respect to each other. Accordingly, the rod lenses may be disposed in an optical path from the distal portion 110 of the endoscope to the proximal portion of the endoscope at least in part within the inner region of the cradle 170. The optical path may provide a path through which collected light travels. In particular, the light from the emitter 120, some of which is reflected from the reflector 140, may be reflected or scattered from the portion of a body cavity illuminated by the solid state emitter 120. The lens 130 may be configured to collect this light The air prism 150 may be configured to redirect light entering the lens 130, such that light will propagate through the cradle 170 parallel to the longitudinal axis (e.g., z-axis) of the cradle 170 and to, for example, the 2D optical detector or an eyepiece, etc.

As illustrated in Fig. 1 and 3, in various embodiments the endoscope may include two electrical lines 180, 182 which extend along the longitudinal length of the cradle 170. The two electrical lines 180, 182 may electrically connect to the solid state emitter 120, e.g., the cathode and anode of an LED. In particular, the two electrical lines 180, 182 may be connected to metallization 183a, 183b (see Fig. 3) on the LED base 160 that is configured to electrically connect to conducting pins or electrical leads on the emitter 120. The two electrical lines 180, 182 may comprise electrical wires such as copper or aluminum wires having insulation thereon. Other types of wiring such as cables may be used.

Referring to Fig. 1 and 3, the space 190 may provide room for the two electrical lines 180, 182 which connect to the cathode and anode of the solid state emitter 120. Other designs, however, are possible.

In some embodiments, the endoscope does not include or use these two lines 180, 182, extending along the longitudinal length of the copper tube 170. Instead in these embodiments, each half 172, 174 of the cradle 170 may be employed as an electrical conductor or line for transmitting electrical power. For example, each half 172, 174 may be electrically connected to the emitter 120, for example, to the respective cathode and anode of the LED. Thus, each half 172, 174, of the cradle 170 may itself comprise the anode or cathode. In some embodiments, the two halves 172, 174 are electrically connected to metallization 183a, 183b on the LED base 160 to form the electrical connection to the emitter 120. In various embodiments, the two halves 172, 174 may be electrically isolated by spaces 190 between the longitudinal edges 210, 212 of the two halves 172, 174. Electrically insulating spacers may also be employed. Although use of the cradle 170 as one or more conductive lines is discussed above as an alternative to using wires 180, 182, in some embodiments, both electrical wires 180, 182 as well as the conducting cradle 170 can be used to transfer electrical signals and/or power between the distal and proximal portions of the endoscope.

In various of these embodiments, the cradle 170 comprises an electrically conductive material, such as copper, thus allowing the cradle 170 to carry electricity. The cradle 170 may thereby operate as an electrical path for providing power or grounding to the solid state emitter 120. In these embodiments, electrical isolation of the cradle 170 and/or halves 172, 174 thereof may be useful to prevent shorts, for example, with an outer tube that fits over the cradle as this outer tube may comprise stainless steel and be conductive. One or more insulating coating may be disposed on the cradle 170, e.g., the outer surface of the cradle, or on the outer tube, e.g., an inner surface of the outer tube, or both. In implementations where the cradle 170 is configured to be electrically conducting and used to carry electricity, an insulating material can be disposed between the distal end 110 and the cradle 170. For example, an insulating material can be disposed between the cover 132 and the cradle 170.

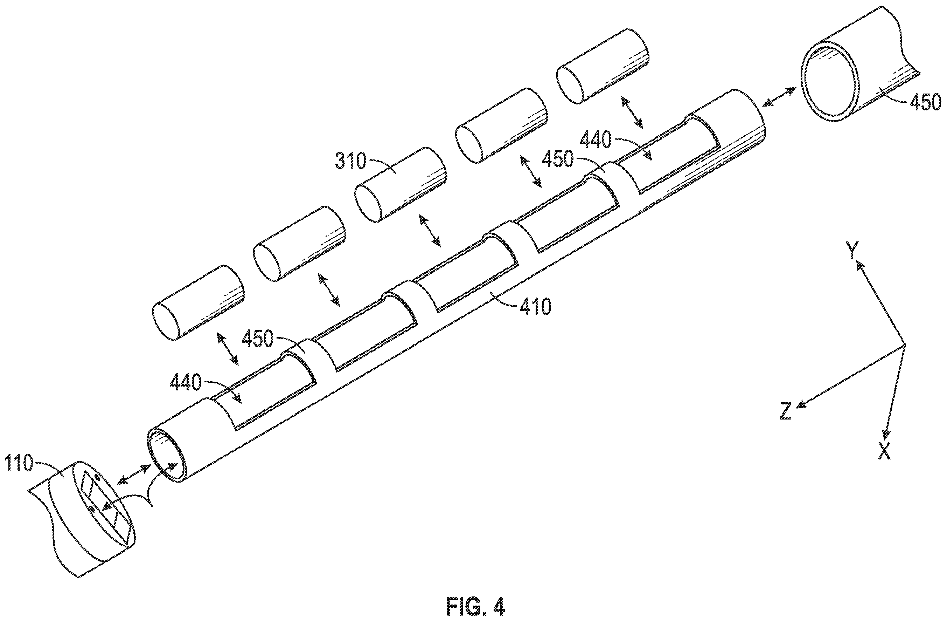

Fig. 4 illustrates some embodiments wherein the endoscope comprises an inner cradle 410 and an outer tube 450. Various aspects of the implementation illustrated in Fig. 4 can be similar to the implementations of endoscopes disclose in U.S. Patent No. 7, 976,462. As discussed above, the inner cradle 410 may comprise a thermally conductive material, such as copper, aluminum, etc. in order to dissipate heat generated by the solid state emitter 120 away from the distal portion 110 of the endoscope. In addition, the outer tube 450 may comprise a thermally conductive material, such as aluminum, stainless steel, or the like. Thus, the outer tube 450 may also conduct heat generated by the solid state emitter 120 away from the distal portion 110 of the endoscope.

In various embodiments, the inner cradle 410 may comprise an electrically conductive material, such as copper, to carry electricity. Accordingly, as discussed above, in some embodiments, the endoscope includes an insulator between the inner cradle 410 and the outer tube 450, in order to prevent shorting out the outer tube 450. In some cases, the inner cradle 410 may include an insulating coating on the exterior of the inner cradle 410. Alternatively, the outer tube 450 may include an insulating coating on the interior of the outer tube 450. In some embodiments, both may be employed. Also insulating spacers between the inner cradle and outer tube may be employed.

As discussed above, with continued reference to Fig. 4, the inner cradle 410 may function as a support structure for one or more lenses 310. In the embodiment shown in Fig. 4, however, the inner cradle 410 has a plurality of slots 440 (five shown) each configured to hold a rod lens 310. The slots 440 may be separated by space portions 450 (four shown) that are each sized and positioned so as to provide proper alignment and longitudinal separation of the rod lenses 310 for suitable relay of an image therethrough. In other words, the spacing between the slots 440 may be defined by spacer portions 450 so as to longitudinally space the rod lenses 310 with respect to each other according to the optical design prescription. In some embodiments, the spacers 450 between the slots provide for structural support and stability to the cradle 410, which comprises thin walls and is susceptible to bending.

In various of these embodiments, during operation, at least the distal portion 110 of the endoscope is inserted into a body cavity. An electrical power signal is provided to the solid state emitter 120, causing the solid state emitter to emit light and generate heat. The thermally conductive cradle 170 dissipates heat away from the distal portion 110 of the endoscope. The thermally conductive cradle 170 may also be electrically conductive, such that the cradle 170 provides an electrical path for providing power or grounding to the solid state emitter 120. Light emitted by the solid state emitter 120 is reflected off an object within the body cavity. A portion of the reflected light is collected by a lens 130. The light is then directed through a plurality of lens elements, such as rod lenses 310, disposed in the cradle 170. Thus, the light propagates from the distal portion 110 of the endoscope to the proximal portion of the endoscope.

Other variations, however, are possible. In certain embodiments, the electrical lines 180, 182 that extend from the proximal end of the endoscope to the emitter 120 have sufficient size coupled with the thermal conductivity thereof, to operate as conduits of thermal energy so as to sufficiently dissipate heat from the emitters and/or distal end of the endoscope. The electrical lines may, for example, comprise cable such as a ribbon cable having sufficient mass and cross-sectional area (in the transverse direction such as x- and/or y- directions) to transfer enough heat away from the emitter to maintain the distal portion of the endoscope at suitable temperatures for insertion into the body. In such embodiments, the electrically conducting cradle 170, 410 within the outer tube 450 may be excluded. The hollow outer tube 450, instead, may have a size such the lenses 310 are held in place within an inner region of the hollow outer tube without the cradle 170, 410.

Various modifications to the implementations described in this disclosure may be readily apparent to those skilled in the art, and the generic principles defined herein may be applied to other implementations without departing from the scope of this disclosure. Thus, the claims are not intended to be limited to the implementations shown herein, but are to be accorded the widest scope consistent with this disclosure, the principles and the novel features disclosed herein.

Certain features that are described in this specification in the context of separate embodiments also can be implemented in combination in a single embodiment. Conversely, various features that are described in the context of a single embodiment also can be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a sub combination or variation of a subcombination.

Similarly, while operations may be described as occurring in a particular order, this should not be understood as requiring that such operations be performed in the particular order described or in sequential order, or that all described operations be performed, to achieve desirable results. Further, other operations that are not disclosed can be incorporated in the processes that are described herein. For example, one or more additional operations can be performed before, after, simultaneously, or between any of the disclosed operations. In certain circumstances, multitasking and parallel processing may be advantageous. Additionally, other embodiments are within the scope of the following claims. In some cases, the actions recited in the claims can be performed in a different order and still achieve desirable results.

## Claims

1. An endoscope, comprising:
a proximal end portion;
a distal end portion (110);
a plurality of lenses (310) disposed in an optical path extending from said distal end portion to said proximal end portion such that an image of an object at the distal end portion can be formed at the proximal end portion;
at least one solid state emitter (120) disposed at the distal end portion, the at least one solid state emitter generating heat when activated; and
a thermally conductive cradle (170) forming an elongate inner open region configured to house said plurality of lenses, said thermally conductive cradle attached to the distal end portion, the thermally conductive cradle configured to dissipate heat generated by the at least one solid state emitter away from the distal end portion,
wherein the thermally conductive cradle is electrically conductive, and
wherein the thermally conductive cradle comprises a first curved section (172) and a second curved section (174);
**characterised in that**:
the thermally conductive cradle comprises copper, wherein said first and second curved sections comprise portions of a right circular cylinder physically separated by a gap along a longitudinal length of the right circular cylinder,
wherein the first and the second sections each have a first longitudinal edge (210) and a second longitudinal edge (212), wherein the first longitudinal edge of the first section and the first longitudinal edge of the second section are separated by a first space (190) and the second longitudinal edge of the first section and the second longitudinal edge the second section are separated by a second space (190),
wherein the first curved section is electrically connected to a first electrode of said at least one solid state emitter and the second curved section is electrically connected to a second electrode of said at least one solid state emitter, and
wherein the first curved section and the second curved section are electrically isolated from each other.

2. The endoscope of Claim 1, wherein said endoscope comprises an arthroscope.

3. The endoscope of any of Claims 1 or 2, wherein the at least one solid state emitter comprises at least one light emitting diode.

4. The endoscope of any of Claims 1 to 3, wherein said plurality of lenses comprise rod lenses having lengths and widths, said lengths being longer than said widths.

5. The endoscope of Claim 1, wherein the first and second sections have substantially equal size.

6. The endoscope of any of Claims 1 to 5, wherein the first and second sections each comprise substantially a half of said right circular cylinder.

7. The endoscope of any of Claims 1 to 6, wherein the first space or the second space is at least 0.5 millimeter.

8. The endoscope of any of Claims 1 to 7, wherein the first longitudinal edge of the first and the second curved sections or the second longitudinal edge of the first and the second curved sections are physically spaced apart by a spacer.

9. The endoscope of any of Claims 1 to 8, further comprising an outer tube surrounding the thermally conductive cradle.

10. The endoscope of Claim 9, wherein said cradle is electrically insulated from said outer tube.

11. The endoscope of any of Claims 1 to 10, wherein the first curved section and the second curved section are electrically isolated from each other by an electrically insulating material.

## Patentansprüche

1. Endoskop mit:
einem nahen Endabschnitt;
einem entfernten Endabschnitt (110);
einer Mehrzahl von Linsen (310), die in einem optischen Weg angeordnet sind, der sich von dem nahen Endabschnitt zu dem entfernten Endabschnitt erstreckt, so dass ein Bild eines Objekts an dem entfernten Endabschnitt an dem nahen Endabschnitt gebildet werden kann;
zumindest einem Festkörperemitter (120), der an dem entfernten Endabschnitt angeordnet ist, wobei der zumindest eine Festkörperemitter Wärme erzeugt, wenn er aktiviert ist; und
einer thermisch leitfähigen Schale (170), die einen länglichen inneren offenen Bereich bildet, der ausgestaltet ist, um eine Mehrzahl von Linsen aufzunehmen, wobei die thermisch leitfähige Schale an dem entfernten Endabschnitt befestigt ist, wobei die thermisch leitfähige Schale ausgestaltet ist, um Wärme weg von dem entfernten Endabschnitt zu zerstreuen, die durch den zumindest einen Festkörperemitter erzeugt wird,;
wobei die thermisch leitfähige Schale elektrisch leitfähig ist, und
wobei die thermisch leitfähige Schale einen ersten gebogenen Abschnitt (172) und einen zweiten gebogenen Abschnitt (174) umfasst;
**dadurch gekennzeichnet, dass**
die thermisch leitfähige Schale Kupfer enthält;
wobei der erste und zweite gebogene Abschnitt jeweils Abschnitte eines geraden Kreiszylinders enthalten, die physikalisch durch eine Lücke entlang einer Längsrichtung des geraden Kreiszylinders getrennt sind,
wobei der erste und zweite Abschnitt jeweils eine erste Längskante (210) und eine zweite Längskante (212) haben, wobei die erste Längskante des ersten Abschnitts und die erste Längskante des zweiten Abschnitts durch einen ersten Raum (190) getrennt sind;
wobei die zweite Längskante des ersten Abschnitts und die zweite Längskante des zweiten Abschnitts durch einen zweiten Raum (190) getrennt sind,
wobei der erste gebogene Abschnitt elektrisch mit einer ersten Elektrode des zumindest einen Festkörperemitters verbunden ist, und wobei der zweite gebogene Abschnitt elektrisch mit einer zweiten Elektrode des zumindest einen Festkörperemitters verbunden ist, und
wobei der erste gebogene Abschnitt und der zweite gebogene Abschnitt voneinander elektrisch isoliert sind.

2. Endoskop nach Anspruch 1, wobei das Endoskop ein Arthroskop umfasst.

3. Endoskop nach einem der Ansprüche 1 oder 2, wobei der zumindest eine Festkörperemitter zumindest eine lichtemittierende Diode umfasst.

4. Endoskop nach einem der Ansprüche 1 bis 3, bei dem die Mehrzahl von Linsen Stablinsen umfassen, die Längen und Breiten haben, wobei die Längen größer als die Breiten sind.

5. Endoskop nach Anspruch 1, bei dem die ersten und zweiten Abschnitte im Wesentlichen die gleiche Größe haben.

6. Endoskop nach Anspruch 1 bis 5, bei dem die ersten und zweiten Abschnitte im Wesentlichen eine Hälfte des geraden Kreiszylinders umfassen.

7. Endoskop nach Anspruch 1 bis 6, wobei der erste Raum oder der zweite Raum 0,5 mm ist.

8. Endoskop nach Anspruch 1 bis 7, bei dem die erste Längskante des ersten und zweiten gebogenen Abschnitts oder die zweite Längskante des ersten und zweiten gebogenen Abschnitts physikalisch voneinander durch einen Abstandhalter getrennt sind.

9. Endoskop nach einem der Ansprüche 1 bis 8, des Weiteren mit einem Außenrohr, dass die thermisch leitfähige Schale umgibt.

10. Endoskop nach Anspruch 9, wobei die Schale elektrisch gegenüber dem Außenrohr isoliert ist.

11. Endoskop nach einem der Ansprüche 1 bis 10, wobei der erste gebogene Abschnitt und der zweite gebogene Abschnitt voneinander durch ein elektrisch isolierendes Material isoliert sind.

## Revendications

1. Endoscope, comprenant :
une partie d'extrémité proximale ;
une partie (110) d'extrémité distale ;
une pluralité de lentilles (310), disposée suivant un trajet optique allant de la partie d'extrémité distale à la partie d'extrémité proximale, de manière à ce qu'une image d'un objet à la partie d'extrémité distale puisse être formée à la partie d'extrémité proximale ;
au moins un émetteur (120) à semi-conducteur, disposé à la partie d'extrémité distale, le au moins un émetteur à semi-conducteur produisant de la chaleur lorsqu'il est activé et
un berceau (170), conducteur de la chaleur, formant une région ouverte intérieure oblongue, configuré pour loger la pluralité de lentilles, le berceau conducteur de la chaleur étant fixé à la partie d'extrémité distale, le berceau conducteur de la chaleur étant configuré pour dissiper de la partie d'extrémité distale de la chaleur produite par le au moins un émetteur à semi-conducteur, dans lequel le berceau conducteur de la chaleur est conducteur de l'électricité et dans lequel le berceau conducteur de la chaleur comprend une première partie (172) incurvée et une deuxième partie (174) incurvée, **caractérisé en ce que**
le berceau conducteur de la chaleur comprend du cuivre,
dans lequel la première et la deuxième parties incurvées comprennent des parties d'un cylindre circulaire droit, séparées physiquement par un intervalle suivant un tronçon longitudinal du cylindre circulaire droit,
dans lequel les première et deuxième parties ont chacune un premier bord (210) longitudinal et un deuxième bord (212) longitudinal,
dans lequel le premier bord longitudinal de la première partie et le premier bord longitudinal de la deuxième partie sont séparés par un premier espace (190) et le deuxième bord longitudinal de la première partie et le deuxième bord longitudinal de la deuxième partie sont séparés par un deuxième espace (190),
dans lequel la première partie incurvée est connectée électriquement à une première électrode du au moins un émetteur à semi-conducteur et la deuxième partie incurvée est connectée électriquement à une deuxième électrode du au moins un émetteur à semi-conducteur et
dans lequel la première partie incurvée et la deuxième partie incurvée sont isolées électriquement l'une de l'autre.

2. Endoscope suivant la revendication 1, dans lequel l'endoscope comprend un arthroscope.

3. Endoscope suivant l'une quelconque des revendications 1 ou 2, dans lequel le au moins un émetteur à semi-conducteur comprend au moins une diode électroluminescente.

4. Endoscope suivant l'une quelconque des revendications 1 à 3, dans lequel la pluralité de lentilles comprend des lentilles à barreau ayant des longueurs et des largeurs, les longueurs étant plus grandes que les largeurs.

5. Endoscope suivant la revendication 1, dans lequel les première et deuxième parties ont des dimensions sensiblement égales.

6. Endoscope suivant l'une quelconque des revendications 1 à 5, dans lequel les première et deuxième parties comprennent chacune sensiblement une moitié du cylindre circulaire droit.

7. Endoscope suivant l'une quelconque des revendications 1 à 6, dans lequel le premier espace ou le deuxième espace est d'au moins 0,5 millimètre.

8. Endoscope suivant l'une quelconque des revendications 1 à 7, dans lequel le premier bord longitudinal de la première et de la deuxième parties incurvées ou le deuxième bord longitudinal de la première et de la deuxième parties incurvées sont physiquement mis à distance par une entretoise.

9. Endoscope suivant l'une quelconque des revendications 1 à 8, comprenant, en outre, un tube extérieur entourant le berceau conducteur de la chaleur.

10. Endoscope suivant la revendication 9, dans lequel le berceau est isolé électriquement du tube extérieur.

11. Endoscope suivant l'une quelconque des revendications 1 à 10, dans lequel la première partie incurvée et la deuxième partie incurvée sont isolées électriquement l'une de l'autre par un matériau isolant électriquement.
